(19) Europäisches Patentamt
European Patent Office
Office européen des brevets

(11) **EP 3 764 365 A1**

(12) # EUROPEAN PATENT APPLICATION

(43) Date of publication:
**13.01.2021 Bulletin 2021/02**

(51) Int Cl.:
***G16H 30/00*** (2018.01)

(21) Application number: **19185178.1**

(22) Date of filing: **09.07.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Bayer Aktiengesellschaft**
**51373 Leverkusen (DE)**

(72) Inventors:
• **Fois, Franco**
  **40789 Monheim (DE)**
• **Liao, Wei**
  **60438 Frankfurt am Main (DE)**
• **Rechsteiner, Daniel**
  **51107 Köln (DE)**

(74) Representative: **BIP Patents**
**c/o Bayer Intellectual Property GmbH**
**Alfred-Nobel-Straße 10**
**40789 Monheim am Rhein (DE)**

(54) **IMPROVEMENTS IN THE RADIOLOGICAL IDENTIFICATION OF CHRONIC THROMBOEMBOLIC PULMONARY HYPERTENSION**

(57) The present invention is concerned with the radiological identification of chronic thromboembolic pulmonary hypertension (CTEPH). The invention relates to a method, a computer system and a computer program product for automatically identifying signs of the presence of CTEPH in a person.

EP 3 764 365 A1

**Description**

The present invention is concerned with the radiological identification of chronic thromboembolic pulmonary hypertension (CTEPH). The invention relates to a method, a computer system and a computer program product for automatically identifying signs of the presence of CTEPH in a person.

Chronic thromboembolic pulmonary hypertension (CTEPH) is a special form of pulmonary hypertension (PH). It is characterized by the infiltration of thrombi into the pulmonary arteries. They clog and constrict the vessels in a complete or partial manner and can be converted into connective tissue. In rare cases, there is the development of pulmonary hypertension with a poor prognosis.

The symptoms of CTEPH are non-specific. In the early stage, dyspnoea and fatigue may occur. The duration of the initial symptoms for diagnosis is on average 14 months, and during the course of this some patients are then already in an advanced stage of the disease. This underlines the need for an exact and timely diagnosis.

A timely diagnosis of CTEPH is also important because there are meanwhile options for the therapy of different forms of manifestation of this disease.

The preferred CTEPH therapy is operative pulmonary endarterectomy (PEA), by means of which it is possible to heal up to 70% of patients. Meanwhile, perioperative mortality at experienced centres is 2-4%. However, about 30-50% of all CTEPH patients are classified as non-operable. For these patients and for patients with persistent pulmonary hypertension after PEA, a medicament-based therapy was authorized for the first time at the beginning of 2014 in the form of Riociguat.

The gold standard for diagnosing or ruling out a case of CTEPH is ventilation/perfusion scintigraphy. The negative predictive value of perfusion scintigraphy is almost 100%; this means that a proper perfusion distribution rules out a case of CTEPH with a probability bordering on certainty.

However, the problem is that CTEPH is comparatively rare. As a result of the rarity and the complex diagnosis and differential diagnosis, CTEPH is underdiagnosed.

There is therefore a need for an early and uncomplicated identification of signs of the presence of CTEPH.

According to the invention, this object is achieved by the subjects of Claims 1, 9 and 14. Preferred embodiments can be found in the dependent claims and in the present description.

The present invention firstly provides a method for identifying signs of the presence of CTEPH in a person, comprising the following steps:

- receiving and/or retrieving one or more computed tomography images of the thorax of the person;

- analysing the one or more computed tomography images by:

    a) segmenting the cardiac region to identify the left ventricle, the right ventricle and the interventricular septum,

    b) determining a 2D slice image with the maximum right ventricle diameter by rotating a plane throughout the main axis of an ellipsoid fitted to the interventricular septum;

- determining at least one feature selected from the group of ratio of the diameters of the right ventricle and left ventricle, and degree of curvature of the interventricular septum based on the 2D slice image as determined in step b) which indicate the presence of CTEPH;

- calculating a probability of the presence of CTEPH on the basis of the at least one feature ascertained;

- communicating a message to the person and/or another person for further assessment of the case if the probability is above a defined threshold;

where the steps mentioned run automatically as background processes in one or more computer systems.

The present invention further provides a computer system for identifying signs of the presence of CTEPH in a person, comprising:

- means for the automatic receipt and/or retrieval of one or more computed tomography images of the thorax of the person;

- means for the automated analysis of the one or more computed tomography images by:

    a) segmenting the cardiac region to identify the left ventricle, the right ventricle and the interventricular septum,

b) determining a 2D slice image with the maximum right ventricle diameter by rotating a plane throughout the main axis of an ellipsoid fitted to the interventricular septum;

- means for the automated identification of at least one feature selected from the group of ratio of the diameters of the right ventricle and left ventricle, and degree of curvature of the interventricular septum based on the 2D slice image as determined in step b) which indicate the presence of CTEPH;

- means for automatically calculating a probability of the presence of CTEPH on the basis of the features ascertained;

- means for the automated communication of a message to the person and/or another person for further clarification of the finding.

The present invention further provides a computer program product comprising a data carrier on which there is stored a computer program which can be loaded into the memory of a computer system, where it causes the computer system to execute the following steps:

- receiving and/or retrieving one or more computed tomography images of the thorax of the person;

- analysing the one or more computed tomography images by

a) segmenting the cardiac region to identify the left ventricle, the right ventricle and the interventricular septum,

b) determining a 2D slice image with the maximum right ventricle diameter by rotating a plane throughout the main axis of an ellipsoid fitted to the interventricular septum;

- determining at least one feature selected from the group of ratio of the diameters of the right ventricle and left ventricle, and the degree of curvature of the interventricular septum based on the 2D slice image as determined in step b) which indicate the presence of CTEPH;

- calculating a probability of the presence of CTEPH on the basis of the features ascertained;

- communicating a message to the person and/or another person for further assessment of the finding if the probability is above a defined threshold

where the steps mentioned run automatically as background processes on the computer system.

The individual elements which characterize the method according to the invention, the computer system and the computer program product are explained in more detail below. This explanation does not make a distinction between the individual subjects of the invention (method, computer system, computer program product). Instead, the following descriptions apply analogously to all subjects of the invention, irrespective of their context.

In the identification of signs of the presence of CTEPH in a person, the present invention focusses on the automated image analysis of computed tomography images of the thorax of the person.

Computed tomography (CT) is an X-ray method which depicts the human body in cross-sectional images (sectional imaging method). Compared to a conventional X-ray image, on which usually only coarse structures and bones are identifiable, CT images also capture in detail soft tissues with small differences in contrast. An X-ray tube generates a so-called X-ray fan beam, which penetrates the body and is attenuated to varying degrees within the body owing to the various structures, such as organs and bones. The receiving detectors opposite the X-ray emitter receive the signals of varying strength and forward them to a computer, which puts together cross-sectional images of the body from the received data. Computed tomography images (CT images) can be observed in 2D or else in 3D. For better differentiability of structures within the body of the person (e.g. vessels), a contrast agent can be injected into a vein before the generation of CT images.

Computed tomography is a commonly used method in the diagnosis of heart and lung diseases. Preferably, the CT images are multidetector CT images.

So-called multidetector CT (MDCT) refers to the newest generation of computed tomographs, having been available in clinical radiology since 1998. Multidetector CT is widely available and distinguished by a high, virtually isotropic resolution (pixel size 0.5-1 mm in each spatial direction), and this makes it possible to view the CT images in any spatial plane. The examination time varies between 1 and 10 seconds, giving rise to images virtually free of artefacts, even when there is dyspnoea or lack of patient cooperation. The latest MDCT scanners are equipped with "dual-energy" technology, in which two different energies/tube voltages are used simultaneously. Owing to the energy dependence of absorption, it is possible to highlight certain tissue properties, for example the distribution of iodine after administration of contrast agent as surrogate for regional blood circulation.

A crucial criterion in the present invention is the automation. As already set out, CTEPH is a rare condition which is underdiagnosed. Non-detection of this condition can have fatal consequences for the patient. Therefore, according to the present invention, computed tomography images of the thorax are analysed in an automated manner for signs of the presence of CTEPH. What is meant here by "automated" is that no human intervention at all is required. According to the invention, a computer program is thus installed on a computer system that has access to computed tomography images of the thorax, runs as a background process, and analyses the images in an automated manner for signs of the presence of CTEPH. A background process refers to a process that does not act directly with the user and hence acts asynchronously to the user interface.

In a first step of the method (and/or as part of the computer system, computer program product), one or more computed tomography images of the thorax of a person are received or retrieved. Customarily, a CT image is a data set, by means of which the structures of the thorax of the person can be depicted three-dimensionally. Thus, a CT image customarily represents the thorax of the person at the time of recording of the computed tomogram. Multiple CT images can e.g., represent the heart and/or pulmonary region of the person at different times; on the basis of these multiple CT images, it is possible therefore to identify temporal changes in the tissue structures and thus, for example, to examine a progression of a disease. However, it is also conceivable that the multiple CT images are CT images representing different regions of the thorax.

As already described, computed tomography is a customary method for the diagnosis of heart and lung diseases. It is therefore conceivable to examine CT images already present in a database for the presence of signs of CTEPH. In one embodiment of the present invention, CT images present in one or more databases are retrieved and are analysed for the presence of signs of CTEPH. For example, this can occur at regular intervals. It is, for example, conceivable to carry out at regular intervals, for example every day or every week, a search for new CT images in the databases in which CT images are customarily deposited and to retrieve the new CT images for an image analysis. However, the retrieval can also take place irregularly. The retrieval can also be triggered by an event, for example by the storing of a new CT image. Preferably, the retrieval of new CT images is done in an automated manner.

It is also conceivable to carry out the analysis according to the invention for the presence of signs of CTEPH as a kind of standard analysis for each CT image generated from the thorax of a person. Accordingly, in a further preferred embodiment of the present invention, a CT image generated from the thorax of a person is, after its generation, immediately and automatically subjected to an image analysis according to the invention. In such a case, a computer system oriented to generating a relevant CT image can be configured such that it forwards the CT image to the image analysis according to the invention. The components executing the image analysis receive the CT image.

In a further step of the method (and/or as part of the computer system, computer program product), an automated analysis of the one or more CT image takes place (preferably more than one CT image). The analysis is carried out by:

a) segmenting the cardiac region to identify the left ventricle, the right ventricle and the interventricular septum,

b) determining a 2D slice image with the maximum right ventricle diameter by rotating a plane throughout the main axis of an ellipsoid fitted to the interventricular septum.

Image-based segmentation techniques of the cardiac region consist of thresholding, region-growing, clustering, pixel or voxel classification, and active contour or surface (Peng et al, A review of heart chamber segmentation for structural and functional analysis using cardiac magnetic resonance imaging, 2016, 29:155-195). In recent years, cardiac segmentation methods are based on deep learning which can be roughly divided into two classes: 2D methods which segment each slice independently and 3D methods, which segment multiple slices together as a volume (Qiao Zheng et al., 3D consistent robust segmentation of cardiac images by deep learning with spatial propagation, IEEE Trans. On Medical Imaging, 2018, hal-01753086).

In particular, segmentation of the cardiac region (referred to above as step a) includes

- producing a binary segmentation of left and right ventricles of the cardiac region,

- resampling the binary segmented image(s) to an isotropic spacing if required,

- thresholding the image(s) to segment the left ventricle and the right ventricle into two separate binary images,

- distance transforming each binary image and intersecting the images,

- identifying the left and right ventricle, and the interventricular septum as the intersection volume between the left and the right ventricle.

Binary segmentation of left and right ventricles of the cardiac region refers in particular to 3D CT images marked by different grey values. The left and right ventricles are in particular coded by different grey values. Resampling of the binary segmented images to an isotropic spacing is usually required as 3D CT images contain voxels for which the spacing in the three spatial directions is not uniform. Resampling refers to process step wherein an isotropic spacing in all direction of the 3D images is achieved. In particular, a spacing value of equal or below 2.5 mm, more particularly equal or below 2.0 mm is targeted. Resampling refers preferably also to the extraction of superfluous tissue such as the interventricular septum e.g., with morphological operators.

In the thresholding step both ventricles are segmented into two new distinct images. In particular, this step also includes an object analysis step in both images to detect the geometric region enclosing the left and the right ventricles followed by a union of the regions. The distance transforming step results in grey level images that look similar to the input images, except that the distance of a voxel from the edge of an object is coded as a grey value: the higher the grey value, the higher the distance. The distance-transformed images are intersected, preferably by using a thresholding step to from a thick, intersecting border around the both ventricles. Finally, the intersection volume is extracted defining the new interventricular septum.

The step b) of the method (and/or as part of the computer system, computer program product) refers to determining a 2D slice image with the maximum right ventricle diameter by rotating a plane throughout the main axis of an ellipsoid fitted to the interventricular septum.

A basis for determining the appropriate 2D slice image is the ellipsoid fitting to the interventricular septum and the determination of a preferred direction and a geometrical characterization of the interventricular septum.

The ellipsoid is a special case of a surface of second order. Such a surface is described by the following implicit function:

$$f(x,y,z) = a_{11}x^2 + a_{22}y^2 + a_{33}z^2 + 2a_{12}xy + 2a_{13}xz + 2a_{23}yz + 2b_1x + 2b_2y + 2b_3z - 1 = 0 \quad (1)$$

Or in matrix notation:

$$f(\vec{r}) = \vec{r}^T A \vec{r} + \vec{b}\vec{r} - 1 = 0 \quad (2)$$

With:

$$A = \begin{bmatrix} a_{11} & a_{12} & a_{13} \\ a_{12} & a_{22} & a_{23} \\ a_{13} & a_{23} & a_{33} \end{bmatrix}, \quad \vec{b} = \begin{bmatrix} b_1 \\ b_2 \\ b_3 \end{bmatrix} \quad and \; \vec{r} = \begin{bmatrix} x \\ y \\ z \end{bmatrix} \quad (3)$$

Equation (1) describes an ellipsoid, when the following condition is fulfilled:

$$k \, det_2 - det_1^2 > 0 \quad (4)$$

Where:

$$det_1 = a_{11} + a_{22} + a_{33} \quad (5)$$

$$det_2 = a_{11}a_{22} + a_{22}a_{33} + a_{11}a_{33} - a_{33}a_{33} - a_{12}a_{12} - a_{13}a_{13} \quad (6)$$

$$k \sim 4 \quad (7)$$

When fitting the surface of second order (equation 1) to the marginal points of the interventricular septum, the ellipsoid condition (equation 6) is in the majority of cases not fulfilled. In some cases a hyperboloid can be fitted. In order to address this issue a constraint condition can be set to force the fitting of the ellipsoid to the interventricular septum (method of Lagrange multiplier). In a preferred embodiment, however, an ellipsoid is pre-fitted to the left ventricle and the centroid of this ellipsoid is used as a constraint condition for fitting a second ellipsoid to the interventricular septum. The left ventricle is in most cases almost rotation symmetric and the fitted parameters of the surface of second order (equation 1) fulfils the condition of the ellipsoid condition (equation 6).

The ellipsoid fitted to the interventricular septum is preferably identified by:

- fitting an ellipsoid to the left ventricle and calculating the centroid of the ellipsoid,

- using the centroid to fit an ellipsoid to the marginal points of the interventricular septum,

- analysing the orientation of the ellipsoid fitted to the interventricular septum.

Analysis of the orientation of the ellipsoid fitted to the interventricular septum preferably includes

- analysing the eigenvalues and eigenvectors of the ellipsoid fitted to the interventricular septum to determine the orientation in particular the Euler angles and the main axis of the ellipsoid.

The centroid of the ellipsoid fitted to the left ventricle is expressed by the following equation 8:

$$\vec{x_0}^T A = -\vec{b} \tag{8}$$

Where:

$$\vec{x_0} = coordinates\ of\ centroid, A\ and\ \vec{b}\ see\ eq\ (3) \tag{9}$$

Equation 2 then simplifies to the following expression:

$$(\vec{r} - \vec{x_0})^T B(\vec{r} - \vec{x_0}) = 1 \quad with \quad b_{ij} = \frac{a_{ij}}{K} \quad and \quad K = 1 + \vec{x_0}^T A \vec{x_0} \tag{10}$$

The radius of the ellipsoid and the coefficients of the rotation matrix are obtained through an eigenvalue analysis of Matrix B:

$$radius\ H_i = \frac{1}{\sqrt{\lambda_i}}, with\ \lambda_i\ as\ eigenvalue,\ i = 1 \dots 3 \tag{11}$$

$$R_{ij} = (\vec{ev_i})_j \quad with\ \vec{ev_i}\ as\ the\ eigenvector\ i,j = 1 \dots 3 \tag{12}$$

The rotation matrix is expressed as a function of the Euler angles $\Phi$, $\vartheta$, and $\psi$.

$$R = \begin{bmatrix} \cos\vartheta\cos\phi & \sin\psi\sin\vartheta\cos\phi - \cos\psi\sin\phi & \cos\psi\sin\vartheta\cos\phi + \sin\psi\sin\phi \\ \cos\vartheta\sin\phi & \sin\psi\sin\vartheta\sin\phi + \cos\psi\cos\phi & \cos\psi\sin\vartheta\sin\phi - \sin\psi\cos\phi \\ -\sin\vartheta & \sin\psi\cos\vartheta & \cos\psi\cos\vartheta \end{bmatrix} \tag{13}$$

The Euler angles are calculated from equation 12 and 13.

In order to determine the 2D slice image with the maximum right ventricle diameter preferably a 3D image is rotated in the rage of [-90, +90] around the main (middle) axis of the ellipsoid fitted to the interventricular septum.

As a next step the method (and/or as part of the computer system, computer program product) relates to the determination of at least one feature selected from the group of ratio of the diameters of the right ventricle and left ventricle, and the degree of curvature of the interventricular septum based on the 2D slice image as determined in the previous step b) which indicate the presence of CTEPH.

The determination of the ratio of the diameters of the right ventricle and left ventricle preferably includes

- identifying the diameters of the left and right ventricle by a measuring the distance of the ventricle boundaries perpendicularly to the main axis of the ellipsoid fitted to the interventricular septum in the 2D slice image as determined in step b) and calculating the ratio of the diameters.

The ratio of the diameters of right ventricle and left ventricle (RV/LV ratio) (see, for example, Gonzales G et al. PLoS ONE 10(5): e0127797) is a feature that can indicate the presence of CTEPH. A value of 0.9 or more in the RV/LV diameter ratio is an indication of the presence of CTEPH.

The determination of the degree of curvature of the interventricular septum based on the 2D slice image as determined in step b) preferably includes

- skeletonizing the interventricular septum on the 2D slice image,

- computing the centerline of the interventricular septum,

- fitting a polynomial of 3th order to the centerline and identifying local maxima of the polynomial.

The determination of the degree of curvature of the interventricular septum requires the extraction of the centerline. This is preferably done by applying a skeleton operator. In some cases the skeleton image may contain additional branches. These branches can be excluded with minimum-path, graph based approaches. Then, a polynomial of higher order is fitted to the centerline. It has turned out, that a polynomial of the 3th leads to best results. The degree of curvature of the interventricular septum is determined around the local maxima of the polynomial, these points are identified by computing the first derivative.

The degree of curvature of the interventricular septum (see, for example, D.A. Moses et al., Quantification of the curvature and shape of the interventricular septum; Magnetic Resonance in Medicine, Vol. 52 (1), 2004, 154-163 and F. Haddad et al.: Septal Curvature is a marker of hemodynamic, anatomical, and electromechanical ventricular interdependence in patients with pulmonary arterial hypertension, Echocardiography Vol. 31(6) 2014, 699-707).

Preferably, the method (and/or as part of the computer system, computer program product) takes into account at least one additional feature indicative for CTEP selected from the group of volumes of the right ventricle and left ventricle, ratio of the volumes of the right ventricle and the left ventricle, ratio of the diameters of the pulmonary artery and the aorta at the level at which the pulmonary artery branches off, presence and/or severity of stenoses, presence and/or severity of mosaic perfusion, presence and/or severity of ground glass opacity.

The ratio of the diameters of the pulmonary artery and the aorta (PA:A ratio) is determined at the point at which the pulmonary artery branches off (see, for example, A.S. Iyer et al.: CT scan-measured pulmonary artery to aorta ratio and echocardiography for detecting pulmonary hypertension in severe COPD, CHEST 2014, Vol. 145(4), 824-832). A PA:A ratio of 0.7 or greater is a further indication of the presence of CTEPH. Characteristic vascular features are, for example, the lack of contrast agent in the distal vessel sections in the case of total obstruction or the formation of rope-ladder thrombi, meshes, stenoses and partial obstructions. The CTEPH-specific parenchymal features include scars, mosaic perfusion, ground glass opacity and bronchial anomalies. The scars arise as a result of infarctions owing to the occlusion of pulmonary vessels that are usually localized in the lower segments. Mosaic perfusion consists of regions of different density resulting from regions of irregular hypo- and hyperperfused regions caused by embolic occlusions, vascular rearrangement of the distal vessels and compensation mechanisms. Hypoperfused regions are observed particularly in the distal direction from the occluded vessels since blood flow and hence the concentration of the contrast agent are reduced in these regions. Hyperdense areas are usually apparent in regions that are now hyperperfused owing to the redistribution and stand out as ground glass opacity. The latter and further characteristic features are described in the literature (see, for example, J. E. Leifheit, Characterization of ground glass opacity in patients with chronic thromboembolic pulmonary hypertension compared to pulmonary hypertension of other WHO classification using Dual Energy CT [in German], Inaugural Dissertation for Attaining the Degree of Doctor of Medicine at the Justus Liebig University of Giessen, 2017).

It is conceivable that, in the image analysis, no at least one CTEPH-specific feature is found in the CT images examined. In such a case, it is possible to store in a database, in relation to the relevant CT image or in relation to the person from whom the CT image was generated, an item of information indicating that no at least one CTEPH-specific feature were identified in the CT image.

If at least one feature indicating the presence of CTEPH was found, it is possible to store in a database, in relation to the relevant CT image or in relation to the person from whom the CT image was generated, an item of information indicating that at least one CTEPH-specific feature was identified in the CT image.

In a further step of the method (and/or as part of the computer system, computer program product), a calculation of a probability of the presence of CTEPH is effected on the basis of the characteristic feature(s) ascertained. A value of 100% indicates that the patient is suffering from CTEPH; a value of 0% indicates that CTEPH can be ruled out.

The probability can be calculated by following many different approaches. It is conceivable, for example, that the ratio of the diameters of the right ventricle and left ventricle and/or degree of curvature of the interventricular septum as ascertained with the analysis described above is/are compared with predefined values. It is also conceivable, for example, that the one or more CT images are examined for the presence of a number of characteristic features. The probability can be calculated from the sum of the number of features found divided by the sum of the number of features tested (P = (sum of features found)/(sum of features tested)). In such an approach, all features are of equal value. Alternatively, it is conceivable that the individual features are weighted with a factor, such that features that are more likely to indicate CTEPH are given a higher value in the probability function than features that are less specific. It is also conceivable that the severity of a feature is determined; where the severity indicates the extent in respect of the presence of CTEPH. The higher the degree (the extent), the clearer the manifestation of the feature and the higher the probability of the presence of CTEPH. It is also conceivable that one or more decision trees or regression trees are run; it may be the case that one feature indicates CTEPH only in combination with another feature. Further methods and combinations of methods of ascertaining the probability are conceivable.

If the probability is above a defined threshold, a message is generated that gives information that the person who was the subject of the CT images has a defined probability of suffering from CTEPH and therefore further examinations should be undertaken to clarify the finding. The threshold value may, for example, be between 20% and 70%. It is preferably above 20% and below 51%.

According to the invention, a message that the person should be subjected to a further diagnostic procedure in order to confirm CTEPH or to reliably rule it out is then communicated. Said message can, for example, be addressed to the person from whom the relevant CT image originates. However, it can also be addressed to his/her physician or hospital carer or to another person who is in contact with the person for whom there are signs of CTEPH. The communication of the message can be a text message (e-mail, SMS, etc.) or voice message.

The invention is described in detail hereinafter with reference to figures and examples, without wishing to restrict the invention to the features or combinations of features described and shown.

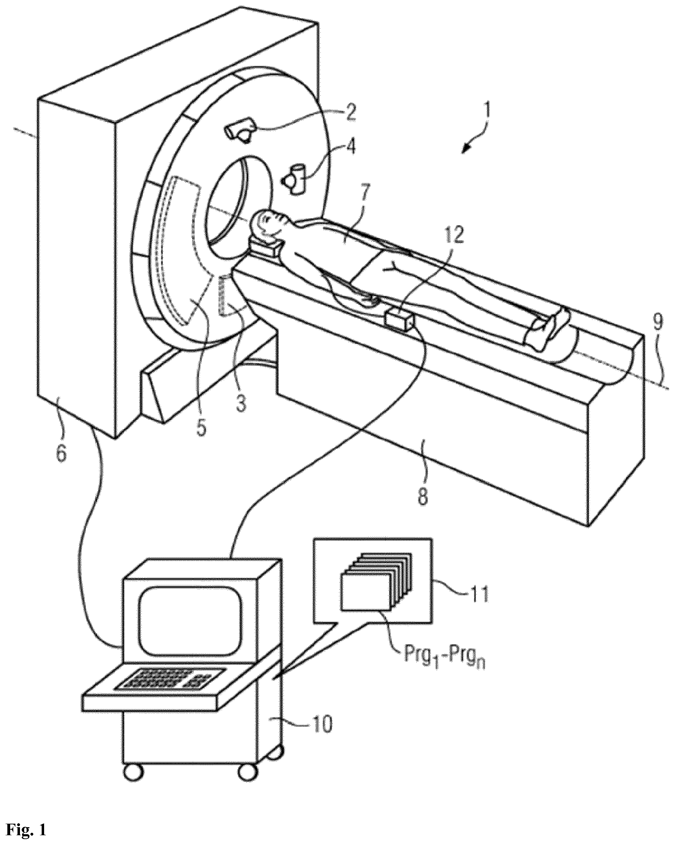

Figure 1 shows, by way of example, one embodiment for the implementation of the system according to the invention.

Figure 1 shows a CT system 1 executed as a twin-focus detector system. It has a first x-ray tube 2 with an opposite detector and a second x-ray tube 4 with a further opposite detector 5. Both focus/detector systems 2, 3 and 4, 5 are arranged in a gantry housing 6 on a gantry which rotates about a system axis 9 and is not visualized here. The patient 7 is on a longitudinally movable patient bed 8. Before the scanning of the patient 7, to improve the contrast of a CT image reconstructed from the detector output data, a contrast agent is administered to the patient 7 by means of a contrast agent injector 12. The control of the overall CT system and if necessary also the evaluation of the detector data and the reconstruction of the CT image as section images or volume data can be effected by a control and computing unit 10. This control and computing unit 10 has a memory 11 which stores, as well as the detector output data measured, computer program data Prg1-Prgn that are executed in operation and essentially assume the function of controlling the system and the evaluation of the data.

In one embodiment of the present invention, the computer program according to the invention runs as a background process on the control and computing unit 10. It analyses the section images or volume data for the presence of CTEPH indications. If CTEPH indications are identified, and a calculated probability of the presence of CTEPH is above a defined threshold, the computer program according to the invention creates a message on the screen of the control and computing unit 10 that informs the radiologist that there is a suspicion of CTEPH.

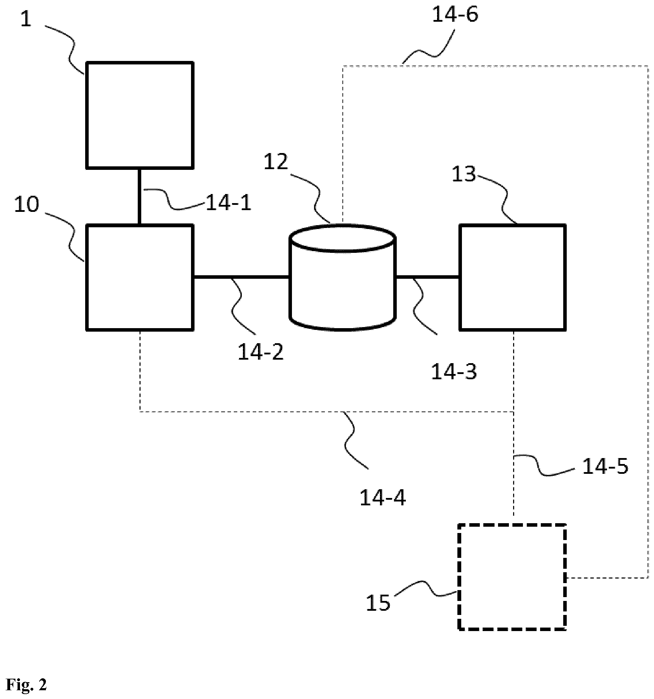

Figure 2 shows a schematic of a further embodiment for the implementation of the system according to the invention.

The CT system 1 is connected via the connection 14-1 to the control and computing unit 10. The control and computing unit 10 controls the CT system 1 and evaluates the detector data and reconstructs the CT image as section images or volume data. The section images and volume data are stored in a database 12 to which the control and computing unit 10 is connected via the connection 14-2. It is also conceivable that the database is part of the control and computing unit 10. The computer system 13 can also access the database 12 via the connection 14-3. The computer program according to the invention is running on the computer system 13. It is configured such that it analyses the CT images of a human thorax stored in the database 12 for indications of the presence of CTEPH. If no indications are identified, corresponding information relating to the CT images is stored. If indications of the presence of CTEPH are identified, the corresponding information relating to the CT images is likewise stored.

The computer program installed and running on the computer system 13 is configured such that, on the basis of the features ascertained that indicate CTEPH, a probability of the presence of CTEPH is calculated. If this probability is above a defined threshold, the computer program creates a message that CTEPH could be present.

The computer program installed and running on the computer system 13 may be configured such that it displays the communication as to the presence of CTEPH indications on a screen which is part of the computer system 13. It is also conceivable that the computer program is configured such that it transmits a message of the presence of CTEPH indications via the connection 14-4 to the control and computing unit 10, via which the message is then displayed, for example on a screen. It is also conceivable that the computer system 10 draws the information as to whether CTEPH indications are present directly from the database 12. It is also conceivable that the computer program is configured such that it transmits a message of the presence of CTEPH indications via the connection 14-5 to a further computer system 15, via which the message is then displayed, for example via a screen. It is also conceivable that the computer system 15 draws the information as to whether CTEPH indications are present from the database 12 via the connection 14-6. The dotted components in Fig. 2 are optional. The connections 14-1, 14-2, 14-3, 14-4, 14-5 and 14-6 may be cable connections, glass fibre-based connections and/or wireless connections (e.g. via radio).

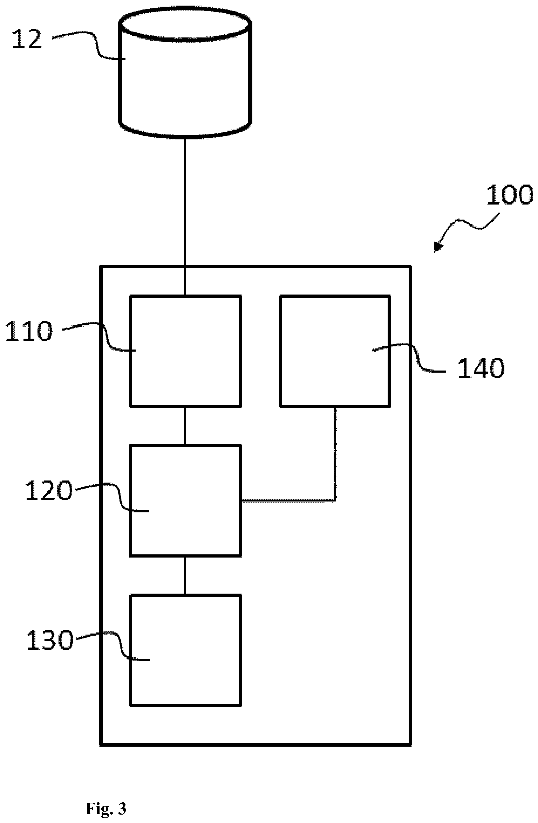

Fig. 3 shows a schematic view of an embodiment of the computer system 100 according to the invention.

The computer system 100 is connected to a database 12 on which the computed tomography images of a human thorax are stored. It is also conceivable that the database 12 is part of the computer system 100. The computer system 100 comprises a receiver unit 110 with which the computed tomography images can be received or retrieved. The computer system 100 comprises a control and computing unit 120 by which the computed tomography images can be analysed and by which features in the computed tomography images that indicate the presence of CTEPH can be recognized. The computer system 100 comprises a computing and checking unit 130 by which a probability of the presence of CTEPH can be calculated and by which it can be checked whether the probability is above a defined threshold. The computing and checking unit 130 may be part of the control and computing unit 120. The computer system 100 comprises an output unit 140 by which a communication as to the result of the analysis can be displayed to a person or transmitted to a person.

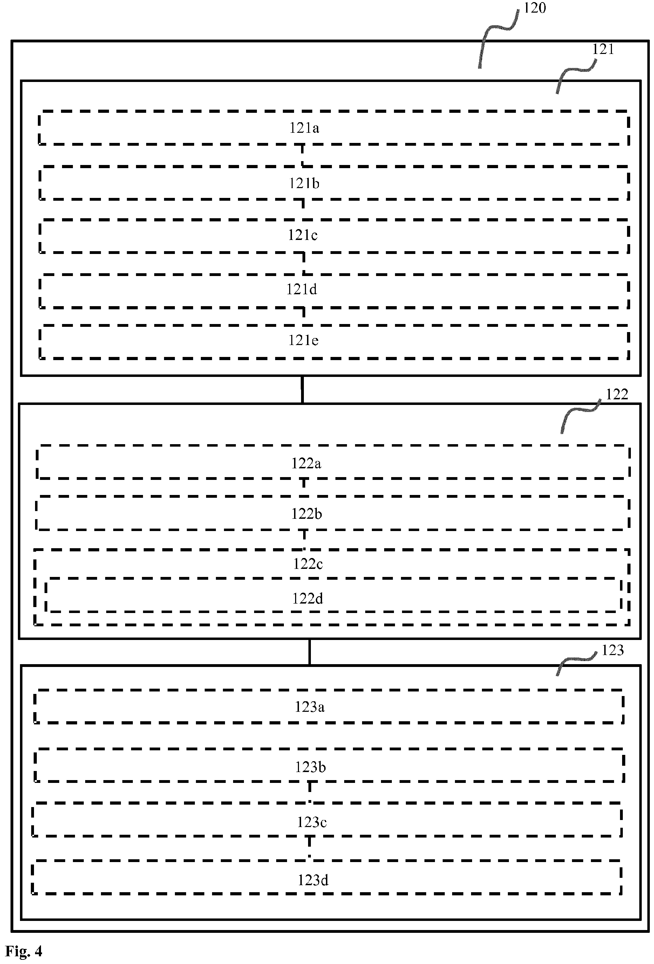

Fig. 4 shows a schematic view of an embodiment of the control and computing unit 120 by which the computed tomography images can be analysed and by which features in the computed tomography images that indicate the presence of CTEPH can be recognized. The dotted components in Fig. 4 are preferred embodiments.

The segmenting (121) of the cardiac region to identify the left ventricle, the right ventricle and the interventricular septum is followed by the determination (122) of a 2D slice image with the maximum right ventricle diameter by rotating a plane throughout the main axis of an ellipsoid fitted to the interventricular septum; and the determination (123) of at least one feature selected from the group of ratio of the diameters of the right ventricle and left ventricle, and degree of curvature of the interventricular septum based on the 2D slice image as determined in step (122) which indicate the presence of CTEPH. It is conceivable that the segmenting step (121) includes producing (121a) a binary segmentation of left and right ventricles of the cardiac region; resampling (121b) the binary segmented image(s) to a isotropic spacing if required; thresholding (121c) the image(s) to segment the left ventricle and the right ventricle into two separate binary images; distance transforming (121d) each binary image and intersecting the images, and identifying (121e) the left and right ventricle, and the interventricular septum as the intersection volume between the left and the right ventricle. It is further conceivable that the determination step (122) includes fitting (122a) an ellipsoid to the left ventricle and calculating the centroid of the ellipsoid; using (122b) the centroid to fit an ellipsoid to the marginal points of the interventricular septum; analysing (122c) the orientation of the ellipsoid fitted to the interventricular septum. The orientation step (122c) preferably includes analysing (122d) the eigenvalues and eigenvectors of the ellipsoid fitted to the interventricular septum to determine the orientation in particular the Euler angles and the main axis of the ellipsoid. The determination step (123) includes identifying (123a) the diameters of the left and right ventricle by measuring the distance of the ventricle boundaries perpendicularly to the main axis of the ellipsoid fitted to the interventricular septum in the 2D slice image as determined in step (122). The determination step (123) also includes determination of the degree of curvature of the interventricular septum by skeletonizing (123b) the interventricular septum on the 2D slice image; by computing the centerline of the interventricular septum (123c); and by fitting (123d) a polynomial of $3^{th}$ order to the centerline and identifying local maxima of the polynomial.

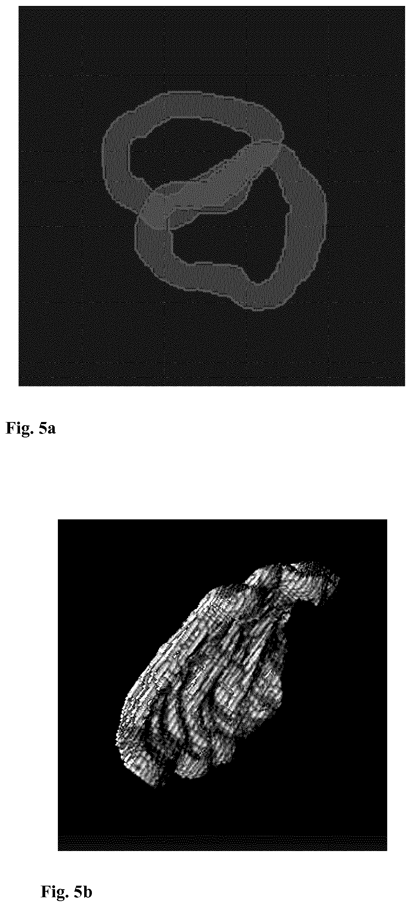

Fig. 5a shows an exemplary picture of the segmentation of the left ventricle, the right ventricle and the intersecting region after distance-transformation and thresholding (step 121d).

Fig. 5b shows an exemplary picture of the intersection volume extracted from the intersecting region as shown in Fig. 5a (step 121e). This intersecting volume represents the interventricular septum.

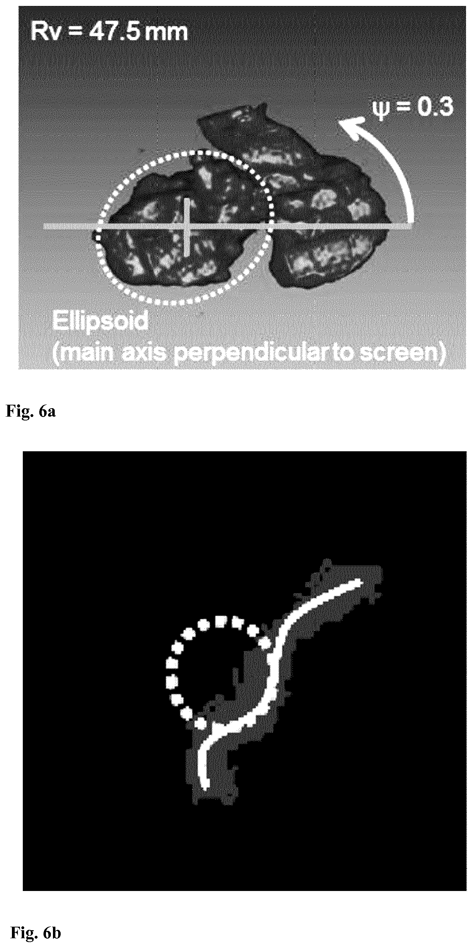

Fig. 6a shows an exemplary picture of the determination of the 2D slice image with the maximum right ventricle diameter (which is 47.5 mm) by rotating ($\psi = 0.3$) a plane throughout the main axis of an ellipsoid fitted to the interventricular septum (step 122).

Fig. 6b shows an exemplary picture of 2D slice image taken for the identification of the centerline of the interventricular septum and the fitting of a polynomial of $3^{th}$ order to the centerline in order to determine the degree of curvature of the interventricular septum (steps 123b to 123d).

**Claims**

1.  Method for identifying signs of the presence of CTEPH in a person, comprising the following steps:

    - receiving and/or retrieving one or more computed tomography images of the thorax of the person;
    - analysing the one or more computed tomography images by:

        a) segmenting the cardiac region to identify the left ventricle, the right ventricle and the interventricular septum,
        b) determining a 2D slice image with the maximum right ventricle diameter by rotating a plane throughout the main axis of an ellipsoid fitted to the interventricular septum;

    - determining at least one feature selected from the group of ratio of the diameters of the right ventricle and left ventricle, and degree of curvature of the interventricular septum based on the 2D slice image as determined in step b) which indicate the presence of CTEPH;
    - calculating a probability of the presence of CTEPH on the basis of the at least one feature ascertained;
    - communicating a message to the person and/or another person for further assessment of the case if the probability is above a defined threshold;

    where the steps mentioned run automatically as background processes in one or more computer systems.

2.  Method according to Claim 1, in which step a) includes:

    - producing a binary segmentation of left and right ventricles of the cardiac region,
    - resampling the binary segmented image(s) to a isotropic spacing if required,
    - thresholding the image(s) to segment the left ventricle and the right ventricle into two separate binary images,
    - distance transforming each binary image and intersecting the images,
    - identifying the left and right ventricle, and the interventricular septum as the intersection volume between the left and the right ventricle.

3.  Method according to any one of Claims 1 to 2, in which step b) includes:

    - fitting an ellipsoid to the left ventricle and calculating the centroid of the ellipsoid,
    - using the centroid to fit an ellipsoid to the marginal points of the interventricular septum,
    - analysing the orientation of the ellipsoid fitted to the interventricular septum,

4.  Method according to Claim 3, in which analysing the orientation of the ellipsoid fitted to the interventricular septum includes

    - analysing the eigenvalues and eigenvectors of the ellipsoid fitted to the interventricular septum to determine the orientation in particular the Euler angles and the main axis of the ellipsoid.

5.  Method according to any one of Claims 1 to 4, in which determination of the ratio of the diameters of the right ventricle and left ventricle based on the 2D slice image as determined in step b) includes the

    - identifying the diameters of the left and right ventricle by measuring the distance of the ventricle boundaries perpendicularly to the main axis of the ellipsoid fitted to the interventricular septum in the 2D slice image as determined in step b).

6.  Method according to any one of Claims 1 to 5, in which determination of the degree of curvature of the interventricular septum based on the 2D slice image as determined in step b) includes

    - skeletonizing the interventricular septum on the 2D slice image,
    - computing the centerline of the interventricular septum,
    - fitting a polynomial of 3th order to the centerline and identifying local maxima of the polynomial.

7.  Method according to any one of the Claims 1 to 6, wherein computed tomography images present in one or more databases are automatically retrieved and forwarded to analysis.

8.  Method according to any one of the Claim 1 to 7, wherein computed tomography images, after their generation, are received from that computer system which generated the computed tomography images and forwarded to analysis.

9.  Computer system for identifying signs of the presence of CTEPH in a person, comprising:

    - means for the automatic receipt and/or retrieval of one or more computed tomography images of the thorax of the person;
    - means for the automated analysis of the one or more computed tomography images by:

        a) segmenting the cardiac region to identify the left ventricle, the right ventricle and the interventricular septum,
        b) determining a 2D slice image with the maximum right ventricle diameter by rotating a plane throughout the main axis of an ellipsoid fitted to the interventricular septum;

    - means for the automated identification of at least one feature selected from the group of ratio of the diameters of the right ventricle and left ventricle, and degree of curvature of the interventricular septum based on the 2D slice image as determined in step b) which indicate the presence of CTEPH;
    - means for automatically calculating a probability of the presence of CTEPH on the basis of the features ascertained;
    - means for the automated communication of a message to the person and/or another person for further clarification of the finding.

10. Computer system according to Claim 9, comprising
    a receiver unit with which the computed tomography images can be received and/or retrieved,
    a control and computing unit by which the computed tomography images can be analysed and by which at least one feature in the computed tomography images that indicate the presence of CTEPH can be recognized,
    a computing and checking unit 130 by which a probability of the presence of CTEPH can be calculated and by which it can be checked whether the probability is above a defined threshold,
    an output unit 140 by which a communication as to the result of the analysis can be displayed to a person or transmitted to a person.

11. Computer system according to any one of Claims 9 and 10, wherein the computer system is configured such that the following processes run in an automated manner as background processes without human intervention:

    - receiving and/or retrieving one or more computed tomography images of the thorax of the person from a database;
    - analysing the one or more computed tomography images by

        a) segmenting the cardiac region to identify the left ventricle, the right ventricle and the interventricular septum,
        b) determining a 2D slice image with the maximum right ventricle diameter by rotating a plane throughout the main axis of an ellipsoid fitted to the interventricular septum;

    - determining at least one feature selected from the group of ratio of the diameters of the right ventricle and left ventricle, and the degree of curvature of the interventricular septum based on the 2D slice image as determined in step b) which indicate the presence of CTEPH;
    - calculating a probability of the presence of CTEPH on the basis of the at least one feature ascertained;
    - communicating a message to the person and/or another person for further assessment of the case if the probability is above a defined threshold.

12. Computer system according to any one of Claims 9 to 11 which is part of a CT system.

13. Computer system according to any of Claims 9 to 11 which is connected to a database on which CT images from a CT system are stored.

14. Computer program product comprising a data carrier on which there is stored a computer program which can be loaded into the memory of a computer system, where it causes the computer system to execute the following steps:

- receiving and/or retrieving one or more computed tomography images of the thorax of the person;
- analysing the one or more computed tomography images by

a) segmenting the cardiac region to identify the left ventricle, the right ventricle and the interventricular septum,
b) determining a 2D slice image with the maximum right ventricle diameter by rotating a plane throughout the main axis of an ellipsoid fitted to the interventricular septum;

- determining at least one feature selected from the group of ratio of the diameters of the right ventricle and left ventricle, and the degree of curvature of the interventricular septum based on the 2D slice image as determined in step b) which indicate the presence of CTEPH;
- calculating a probability of the presence of CTEPH on the basis of the features ascertained;
- communicating a message to the person and/or another person for further assessment of the finding if the probability is above a defined threshold

where the steps mentioned run automatically as background processes on the computer system.

**Fig. 1**

1

14-6

12

13

10

14-1

14-2

14-3

14-4

14-5

15

**Fig. 2**

12

100

110

140

120

130

**Fig. 3**

120

121

121a

121b

121c

121d

121e

122

122a

122b

122c

122d

123

123a

123b

123c

123d

**Fig. 4**

Fig. 5a

Fig. 5b

**Fig. 6a**

**Fig. 6b**

Europäisches
Patentamt

European
Patent Office

Office européen
des brevets

# EUROPEAN SEARCH REPORT

Application Number

EP 19 18 5178

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GERMÁN GONZÁLEZ ET AL: "Automated Axial Right Ventricle to Left Ventricle Diameter Ratio Computation in Computed Tomography Pulmonary Angiography", PLOS ONE, vol. 10, no. 5, 22 May 2015 (2015-05-22), page e0127797, XP055654784, DOI: 10.1371/journal.pone.0127797 * the whole document * | 1-14 | INV. G16H30/00 |
| X | US 2016/260210 A1 (GONZÁLEZ GERMÁN SERRANO [US] ET AL) 8 September 2016 (2016-09-08) * paragraphs [0022] - [0075] * | 1-14 | |
| X | KANAKO K. KUMAMARU ET AL: "Implementation and Performance of Automated Software for Computing Right-to-Left Ventricular Diameter Ratio From Computed Tomography Pulmonary Angiography Images :", JOURNAL OF COMPUTER ASSISTED TOMOGRAPHY, vol. 40, no. 3, 1 January 2016 (2016-01-01), pages 387-392, XP055654787, US ISSN: 0363-8715, DOI: 10.1097/RCT.0000000000000375 * the whole document * | 1-14 | |
| X | SIMISANI TAKOBANA: "Quantification o f Right Ventricular Function in Pulmonary Hypertension using Cardiac PET Images", CARLETON UNIVERSITY, 1 January 2012 (2012-01-01), XP055655157, * the whole document * | 1-14 | TECHNICAL FIELDS SEARCHED (IPC)<br><br>G16H |

-/--

The present search report has been drawn up for all claims

| Place of search | Date of completion of the search | Examiner |
|---|---|---|
| Munich | 23 January 2020 | Díaz de Lezana, C |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

Application Number

EP 19 18 5178

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BENJAMIN H. FREED ET AL: "MR and CT Imaging for the Evaluation of Pulmonary Hypertension", JACC: CARDIOVASCULAR IMAGING, vol. 9, no. 6, 1 June 2016 (2016-06-01), pages 715-732, XP055654791, AMSTERDAM, NL ISSN: 1936-878X, DOI: 10.1016/j.jcmg.2015.12.015 * the whole document * ----- | 1-14 | |

| | | TECHNICAL FIELDS SEARCHED (IPC) |
|---|---|---|

The present search report has been drawn up for all claims

| Place of search | Date of completion of the search | Examiner |
|---|---|---|
| Munich | 23 January 2020 | Díaz de Lezana, C |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

                       
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 19 18 5178

This annex lists the patent family members relating to the patent documents cited in the above-mentioned European search report.
The members are as contained in the European Patent Office EDP file on
The European Patent Office is in no way liable for these particulars which are merely given for the purpose of information.

23-01-2020

| Patent document cited in search report | Publication date | Patent family member(s) | Publication date |
|---|---|---|---|
| US 2016260210 A1 | 08-09-2016 | EP 3074949 A2<br>US 2016260210 A1<br>WO 2015078980 A2 | 05-10-2016<br>08-09-2016<br>04-06-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **QIAO ZHENG et al.** 3D consistent robust segmentation of cardiac images by deep learning with spatial propagation. *IEEE Trans. On Medical Imaging,* 2018
- **GONZALES G et al.** *PLoS ONE,* vol. 10 (5), e0127797
- **D.A. MOSES et al.** Quantification of the curvature and shape of the interventricular septum. *Magnetic Resonance in Medicine,* 2004, vol. 52 (1), 154-163
- **F. HADDAD et al.** Septal Curvature is a marker of hemodynamic, anatomical, and electromechanical ventricular interdependence in patients with pulmonary arterial hypertension. *Echocardiography,* 2014, vol. 31 (6), 699-707
- **A.S. IYER et al.** CT scan-measured pulmonary artery to aorta ratio and echocardiography for detecting pulmonary hypertension in severe COPD. *CHEST,* 2014, vol. 145 (4), 824-832
- **J. E. LEIFHEIT.** Characterization of ground glass opacity in patients with chronic thromboembolic pulmonary hypertension compared to pulmonary hypertension of other WHO classification using Dual Energy CT [in German. *Inaugural Dissertation for Attaining the Degree of Doctor of Medicine at the Justus Liebig University of Giessen,* 2017